# EUROPÄISCHE PATENTSCHRIFT

(11) **EP 2 877 107 B1**
(45) Veröffentlichungstag und Bekanntmachung des Hinweises auf die Patenterteilung: **03.10.2018**
(21) Anmeldenummer: 13735033.6
(22) Anmeldetag: 10.07.2013
(51) Int. Cl.: A61B 17/34

(54) **MEDIZINISCHE EINFÜHRHILFE MIT GEZOGENEM EINFÜHRKANAL**
MEDICAL INSERTION AID HAVING A DRAWN INSERTION DUCT
AUXILIAIRE D'INSERTION MÉDICAL À CANAL D'INSERTION ÉTIRÉ

(30) Priorität: 26.07.2012 DE 102012213205
(43) Veröffentlichungstag der Anmeldung: 03.06.2015
(73) Patentinhaber: Aesculap AG, 78532 Tuttlingen (DE)
(72) Erfinder: SAUTER, Wolfgang, 78603 Renquishausen (DE); DWORSCHAK, Manfred, 78589 Dürbheim (DE); WITTMER, Axel, 32049 Herford (DE)
(74) Vertreter: Winter, Brandl, Fürniss, Hübner, Röss, Kaiser, Polte - Partnerschaft mbB
(86) Internationale Anmeldenummer: PCT/EP2013/064614
(87) Internationale Veröffentlichungsnummer: WO 2014/016123

(56) Entgegenhaltungen:
- DE-U1- 20 309 079
- FR-A1- 2 900 562
- US-A- 5 339 800
- US-A- 5 575 756
- US-A1- 2003 045 834
- US-A1- 2004 230 218
- US-A1- 2009 012 362
- US-A1- 2009 192 444
- US-A1- 2012 053 410
- US-A1- 2012 083 661
- US-B1- 6 733 479

## Beschreibung

Die vorliegende Erfindung betrifft eine Trokarhülse bzw. eine Einführhilfe für chirurgische Instrumente (Trokare, Endoskope, etc.) gemäß dem Oberbegriff des Anspruchs 1. Insbesondere betrifft die Erfindung eine medizinische Einführhilfe mit einem zumindest abschnittsweise gezogenen Einführ- oder Arbeitskanal.

### Hintergrund der Erfindung

Einführhilfen für chirurgische Instrumente werden insbesondere für minimal - invasive Eingriffe am Patientenkörper dazu verwendet, chirurgische Instrumente und/oder Endoskope in einen (ggf. künstlich geschaffenen) Körperhohlraum einzuführen. Der Vorteil derartiger Einführhilfen besteht je nach Einsatzort und -zweck darin, dass entweder nur kleine Zugangsöffnungen durch entsprechende Gewebeschnitte erforderlich sind, um entsprechende Instrumente in den Patientenkörper zu bringen oder dass beispielsweise im Fall einer koloskopischen oder gastroskopischen Anwendung überhaupt keine äußere Verletzung verursacht werden. In soweit werden für die betreffenden Einführhilfen starre, aber auch biegeflexible Rohr- bzw. Schlauchelemente bereitgestellt, die innenseitig zumindest einen Einführkanal vorbestimmten Durchmessers für das Hindurchschieben des chirurgischen Instruments/Endoskops ausbilden.

Eine besondere Form einer solchen Einführhilfe stellt der Trokar dar. Hierbei handelt es sich um ein Instrument, mit dessen Hilfe scharf oder stumpf ein Zugang zu einer Körperhöhle wie dem Bauch- oder Brustraum geschaffen und durch eine Trokarhülse (Tubus) offengehalten wird. Der zunächst in die Trokarhülse eingeführte Obturator ist ein Stift oder Stab, der in dem Tubus axialverschiebbar lagert und dessen Spitze die Tubusöffnung verschließt. Nach Einführen der Obturator - Trokar - Einheit in die Körperhöhle hat der Chirurg dann nach Herausziehen des Obturators aus dem Trokar die Möglichkeit, beispielsweise mit einer Optik (Endoskop) durch den Trokar in die Körperhöhle zu schauen oder mit chirurgischen Greif- und/oder Schneideinstrumenten durch den Trokar hindurch in der Körperhöhle zu operieren.

Die Tuben/Trokare speziell für die Inspektion von Körperhöhlen (Laparoskopie, Thorakoskopie, Arthroskopie, etc.) können an ihrem jeweiligen proximalen, extrakoporalen Endabschnitt einen Ventilmechanismus, einen Anschluss zum Insufflieren (Einblasen) eines Gases oder einer Spülflüssigkeit oder andere Zusatzausstattungen besitzen.

### Stand der Technik

Gattungsgemäße Hülsen sind aus verschiedenen Dokumenten bekannt. So offenbaren insbesondere die Dokumente US 5 575 756 A, US 2012 / 053410 A1, FR 2 900 562 A1, US 2009 / 192 444 A1, US 2003 / 045 834 A1, US 2009 / 012362 A1, US 2004 / 230218 A1 sowie US 2012 / 083661 A1 Vorrichtungen, welche in der Gesamtschau die Präambel des Anspruchs 1 offenbaren.

Aus dem Stand der Technik ist es bekannt, beispielsweise bei konventionellen laparoskopischen Eingriffen üblicherweise Endoskope mit einem (Außen-) Durchmesser von 10mm in Verbindung mit entsprechenden (starren) Trokaren/Trokarhülsen (Tuben) zu verwenden. Über diese sogenannten Optiktrokare wird in der Regel auch die Gasinsufflation zur Erzeugung und/oder Aufrechterhaltung eines Pneumoperitoneums unterhalb der Bauchdecke vorgenommen. Die Toleranzen zur Schaffung eines Ringspalts zwischen der Optik (Endoskop - Außendurchmesser) und dem Trokarhülsen - Innendurchmesser sind normaler Weise so bemessen, dass durch diesen ein ausreichender Gasdurchlass möglich ist, um Gasleckagen aus dem Pneumoperitoneums mindestens auszugleichen und somit die Körperhöhle (unter Gasdruck) ausgedehnt zu halten.

Die Miniaturisierung der Zugänge, insbesondere des Durchmessers der verwendeten chirurgischen und optischen Instrumente einschließlich eines Endoskops beispielsweise in der Laparoskopie bzw. Endoskopie im Allgemeinen, schreitet jedoch immer weiter voran, sodass sogenannte "Single - Port" oder "single - Incision" - Eingriffe als eine Einsatzoption derart verkleinerter Instrumente immer mehr Verbreitung finden. Dabei wird zumeist in der Nabelgrube ein nur ca. 2cm langer Hautschnitt angelegt. Durch diese Inzision werden dann beispielsweise drei 5mm Trokare (Trokarhülsen/Tuben) per einzelne Faszieninzisionen eingebracht.

Diese neuerliche Technik wird demzufolge durch den Einsatz einer 5mm Optik (Endoskop) ermöglicht, was als solches zunächst grundsätzlich kein technisches Problem darstellt, da diese Optiken mittlerweile hinsichtlich der Bild- und Auflösungsqualität also auch der Lichtempfindlichkeit einem 10mm Modell früherer Bauart nahezu entsprechen. Außerdem ist die Lichtstärke von Beleuchtungskörpern (LED's) der letzten Generation sehr hoch und demzufolge die Wärmeentwicklung vergleichsweise niedrig, sodass moderne Optiken bzw. lichtempfindliche Sensoren (CMOS Chips) nicht übergebührlich thermisch beeinträchtigt werden.

Durch die genannte Miniaturisierung treten jedoch neue Probleme an anderen Stellen des vorstehend umschriebenen Einführhilfe - Instrument - Systems auf, die so bislang nicht bekannt waren, zumindest jedoch keine nachteiligen Auswirkungen auf die chirurgischen Arbeitsabläufe hatten. Hierzu zählen u.a. die nachfolgend hervorgehobenen Probleme:

### 1. Unzulängliche Gasinsufflation

Der maximale distale Gasdurchlass bei der Verwendung beispielsweise einer 5mm Optik (Endoskop) in Verbindung mit einem entsprechenden 5mm Trokar (Tubus) erweist sich als grenzwertig niedrig und schränkt die Erzeugung/Aufrechterhaltung eines Pneumoperitoneums erheblich ein. Sofern das Pneumoperitoneum ganz neu aufgebaut werden soll, geht wertvolle Zeit verloren oder es ist überhaupt nicht möglich. Der Grund hierfür besteht prinzipiell darin, dass der Ringspaltquerschnitt zwischen der Trokarhülse und dem darin eingeführten Instrument (Optik) bei einem Instrumentendurchmesser von 5mm (mit entsprechendem Übermaß auf der Trokarhülsenseite) zu klein ist, um einen ausreichenden Gasvolumenstrom zu erreichen, der den Gasleckagestrom wenigstens ausgleicht. D.h., dass in diesem Fall nicht die technischen/konstruktiven Miniaturisierungsmöglichkeiten bezüglich der Optik (Endoskop) sondern einfache strömungsmechanische Gegebenheiten eine Miniaturisierungsgrenze für das Einführhilfe - Instrument - System bilden.

Eine bekannte Konstruktionsvariante sieht diesbezüglich vor, die Trokarhülse an deren distalem Endabschnitt mit einer innenseitigen radialen Einschnürung (Einziehung), abgestimmt auf den Obturator- bzw. Instrumentendurchmesser, auszubilden, um dadurch eine Art Drosselstelle für die Gasströmung zu erzeugen und gleichzeitig eine verbesserte distale Führung des in die Trokarhülse (Tubus) eingeführten Instruments zu erreichen. Vor der innenseitigen radialen Einschnürung (in Richtung proximal) ist das Lumen des Zuführkanals innerhalb des Tubus jedoch um einige Zehntel Millimeter radial aufgeweitert, sodass ein Ringspalt zwischen einem eingeführten Instrument und der Trokarhülse entsteht.

Damit trotz der Anordnung der vorstehend beschriebenen radialen Einschürung bzw. Einziehung am distalen Ende der Trokarhülse genügend Gas über den Trokar in den Körperhohlraum ausströmen kann, sind teilweise kurz (proximal) vor der Einschnürung/Einziehung eine Anzahl von lateralen Austrittsbohrungen in die Trokarhülse eingebracht, aus denen dann Gas in das Pneumoperitoneum seitlich (in Richtung radial) austreten kann. Da jedoch das Gas durch den immer noch engen Ringspalt proximal zu den radialen Austrittsöffnungen strömen muss, bleibt der austretende maximale Gasvolumenstrom grundsätzlich limitiert.

### 2. Relativ stärkere Verschmutzung der Optik

Bei einer 2cm Inzision gemäß vorstehender Beschreibung werden in der Regel kleinere Blutgefäße verletzt, die nach dem Einbringen der Trokare zumindest temporär zu einer geringfügigen Sickerblutung führen. Hierbei sammeln sich mit der Zeit ein oder mehrere Tropfen aus Blut und Gewebeflüssigkeit an der distalen Spitze (distalen Vorderkante) der Trokarhülse an. Wird nun die am distalen Trokarhülsenende herausragende Optik (Endoskop) beispielsweise zur Reinigung der Linse aus der Trokarhülse heraus- bzw. zurückgezogen, was von Zeit zu Zeit notwendig ist, um Sicht - behindernden Niederschlag zu entfernen (dieser bildet sich nach einer gewissen OP-Zeit durch den Einsatz beispielsweise einer HF- oder Ultraschallapplikation), wird der an der Vorderkante anhaftende Blut-/Gewebeflüssigkeitstropfen in die Trokarhülse mit eingesogen und verschmutzt/überzieht diese innenseitig an deren Kanalwand. Wird nun die gereinigte Optik (Endoskop) wieder in die Trokarhülse (mit geringem Ringspaltmaß) eingeführt, wird dadurch die innenseitig anhaftende Flüssigkeit wenigstens anteilig abgestreift und verschmutzt so die Linse sofort wieder zumindest randseitig.

Bei den bislang üblichen 10mm Optiken (Endoskopen) tritt dieses Problem zwar grundsätzlich auch auf, jedoch in deutlich abgeschwächter Form aus den folgenden Gründen:
- Durch das größere Ringspaltmaß zwischen Optik (Endoskop) und Trokarhülse verbleibt bei erneutem Vorschieben der Optik (Endoskop) mehr Flüssigkeit im Ringspalt und gelangt so erst gar nicht auf die Optik (bzw. die Linse).
- Durch die größere Optik (Linse) wird anteilig eine geringere Optik- bzw. Linsenfläche (randseitig) verschmutzt, wobei diese Verschmutzung einen nur geringen Einfluss auf die Leistung der Optik hat.

### Kurzbeschreibung der Erfindung

Angesichts dieser Problematik ist es die Aufgabe der vorliegenden Erfindung, einen ausreichenden Gasdurchfluss zu gewährleisten. Vorzugsweise sollte auch Maßnahmen ergriffen werden, die Optikverschmutzung zu reduzieren. Ein besonderes Ziel ist es hierbei, die vorstehende Aufgabe zu lösen, ohne dass der Außendurchmesser der Trokarhülse wesentlich (oder gar nicht) vergrößert wird und ohne dass das distale Innenlumen der Hülse aufgeweitet wird. Letzteres ist besonders deshalb wichtig, da ansonsten zwischen Obturator (bzw. eingebrachtem Instrument) und dem Innendurchmesser der Trokarhülse ein solches Spaltmaß entsteht, dass in den Ringspalt beispielsweise Fasziengewebe insbesondere beim Einbringen des Trokars / Instruments eingezogen werden könnte.

Die erfindungsgemäße Aufgabe sowie die weiteren Ziele werden durch eine Einführhilfe mit den Merkmalen des Anspruchs 1 gelöst. Vorteilhafte Ausgestaltungen und Weiterbildungen sind Gegenstand der Unteransprüche.

Nach einem ersten (ggf. unabhängig beanspruchbaren) Aspekt der vorliegenden Erfindung hat die medizinische Einführhilfe für chirurgische Instrumente und/oder Optiken (Endoskope) einen Schaft (Trokarhülse/Tubus), der einen Einführkanal (Arbeitskanal) ausbildet oder aufweist, an dessen Innenwand zumindest eine in Kanallängsrichtung orientierte (verlaufende) Nut ausgeformt ist. Die zumindest eine Nut kann dabei gerade in Achsrichtung des Schafts verlaufen oder sich spiral-, zick-zackförmig oder anderweitig (zumindest anteilsmäßig) längserstrecken.

Durch die zumindest eine Nut wird quasi ein lokal radial erweiterter (zusätzlicher) Strömungskanal für das Ein- und/oder Ausbringen eines Gases/Fluids geschaffen, dessen Querschnitt im Wesentlichen unabhängig vom generellen Durchmesser des Zuführkanals und damit vom Ringspaltmaß ist und so einen ausreichenden Gasvolumenstrom gewährleistet. Da die zumindest eine Nut nur partiell eine Materialdünnung am Schaft bedingt, bleibt dessen Stabilitätseigenschaft gegenüber einem Schaft ohne Nut weitgehend erhalten. Eine Verdickung der Schaftwand gegenüber einem nutenlosen Schaft (gemäß dem Stand der Technik) ist nicht oder nur sehr geringfügig notwendig. Schließlich beeinflusst die zumindest eine Nut nicht die Führungsfunktion des Schafts bezüglich des eingeführten chirurgischen Instruments/Optik (Endoskop).

Gemäß einem weiteren ggf. unabhängig zu beanspruchenden Aspekt der vorliegenden Erfindung kann eine Mehrzahl von Nuten vorzugsweise in gleichem Umfangsabstand zueinander an der Innenwand des Einführkanals ausgeformt sein. Dadurch können die Nutenquerschnitte jeweils verkleinert werden, wobei in ihrer Gesamtheit immer noch ein ausreichender Volumenstrom erzeugbar ist. Außerdem wird dadurch die Schwächung der Schaftwand weiter verringert.

Vorteilhaft kann es sein, wenn die zumindest eine Nut oder die Mehrzahl von Nuten vor Erreichen des distalen Schaftendes auslaufen (enden). In diesem Fall kann vermieden werden, dass Fasziengewebe insbesondere beim Einbringen des Trokars / der Einführhilfe in den Patientenhohlraum in die zumindest eine Nut oder Nuten eingezogen wird. Dieses Ergebnis lässt sich optional dadurch noch verbessern, wenn im distalen Endabschnitt des Schafts eine radiale Einschnürung oder Einziehung den Einführkanal vorzugsweise über dessen gesamten Umfang verengt, wobei in diesem Fall die zumindest eine Nut oder die Mehrzahl von Nuten proximal zur Einschnürung / Einziehung oder in einem axialen Mittenabschnitt der Einschnürung / Einziehung (kontinuierlich oder abrupt) auslaufen.

Gemäß einem weiteren, ggf. unabhängigen Aspekt der vorliegenden Erfindung können insbesondere dann, wenn eine radiale Einschnürung / Einziehung am distalen Endabschnitt des Einführkanals vorgesehen ist (nicht darauf beschränkt), eine Anzahl von radialen Durchgangsbohrungen (mindestens Eine) im distalen Endabschnitt des Schafts sowie vorzugsweise in einem proximalen Abstand zur radialen Einschnürung vorgesehen sein, welche den Einführkanal mit der lokalen Schaftumgebung radial (fluid-)verbinden. Auf diese Weise strömt der Gasvolumenstrom nicht oder nur gering an der distalen Schaftspitze aus, sondern wird in die radialen Durchgangsbohrungen umgeleitet, um dann seitlich des Schafts aus dem Zuführkanal (umfassend den Ringspalt sowie die zumindest eine Nut) auszuströmen.

Vorzugsweise sind die Anzahl von radialen Durchgangsbohrungen in Umfangsrichtung im (gleichen) Abstand voneinander angeordnet und weiter vorzugsweise so ausgerichtet und/oder platziert, dass sie in die Nuten an der Innenwand des Einführkanals münden bzw. sich mit diesen schneiden. So kann der Gasvolumenstrom, der durch die Nuten geführt wird, nahezu ungehindert in die Durchgangsbohrungen geführt werden, sodass Druckverluste reduzierbar sind.

Besonders günstig kann es hierbei sein, wenn die Durchgangsbohrungen in Axialrichtung vorzugsweise wechselweise voneinander versetzt sind und dabei zwei oder mehrere (axialbeabstandete) Umfangsreihen bilden. In diesem Fall ist es vorteilhaft, wenn sich die axial versetzt zueinander angeordneten Durchgangsbohrungen in Umfangsrichtung jeweils überlappen. Insbesondere aufgrund der axial versetzten Anordnung der Durchgangsbohrungen lässt sich ein in Umfangsrichtung im wesentlichen geschlossener, flacher sowie radial nach allen Seiten gerichteter Auslass-Gasstrahl erzeugen, der in Abhängigkeit der Gasaustrittsgeschwindigkeit (und damit in Abhängigkeit der Durchgangsbohrungsquerschnitte) wie eine (pneumatische) Sperre oder Schild gegen eine längs der Schaftaußenseite strömende Flüssigkeit (Tropfen) wirken kann. So ist es möglich, bereits im Vorfeld zu vermeiden, dass sich ein Flüssigkeitstropfen an der distalen Vorderkante des Schafts ausbildet, der dann in die zumindest eine Nut eingesaugt wird.

Gemäß der Erfindung weist die medizinische Einführhilfe für chirurgische Instrumente und/oder Optiken (Endoskope) einen Schaft (Trokarhülse/Tubus) auf, der einen Einführkanal (Arbeitskanal) ausbildet oder hat. In einem axialen Mittenabschnitt des Schafts, vorzugsweise im Bereich seines distalen Endes, weist der Schaft an seinem Außenumfang eine radial nach außen vorragende Strom - Sperrkante auf, die sich vorzugsweise ringförmig (weiter vorzugsweise als geschlossener Ring oder in Ringsegmente unterteilt) um den Schaft erstreckt. An dieser Sperrkante sammelt sich die an der Schaftaußenseite ablaufende Flüssigkeit (ggf. zu einzelnen Tropfen) und wird so an einem Weiterlaufen in Richtung distaler Schaftspitze gehindert.

Die Strom - Sperrkante bildet zumindest eine axial sich öffnende Kreisrinne oder -nut mit Öffnungsrichtung nach distal und/oder proximal, und bildet so zumindest eine Nuten- oder rinnenförmige Hinterschneidung die sich axial in proximaler und/oder distaler Richtung öffnet. In anderen Worten ausgedrückt, hat die Strom - Sperrkante in Querschnitt eine L-, U-, V-, X oder H-Form, wodurch sich vorzugsweise zusammen mit der Schaft - Umfangswand zumindest eine (oder zwei Rinnen) zu bilden, die sich entweder in Richtung nach distal, oder in Richtung nach proximal oder gleichzeitig in beide Axialrichtungen (im Fall der X- oder H-Form) öffnen.

Die in Richtung proximal geöffnete Rinne bildet somit eine Art Flüssigkeits - Auffangrinne, in der sich ablaufende Flüssigkeit ansammeln kann. Die Rinnenbreite ist dabei vorzugsweise so gewählt, dass im Rinnenspalt eine Kapillarwirkung entsteht, wodurch die herab (in Richtung distal) laufende Flüssigkeit regelrecht in den Rinnenspalt eingesogen wird. Die in Richtung distal geöffnete Rinne hingegen bildet an ihrer radial äußeren (freien) Kante vornehmlich eine Abtropfkante, von der herab laufende, ggf. zu Tropfen sich formende Flüssigkeit frei abtropfen kann. Letzterer Effekt kann ggf. noch verstärkt werden, indem radial sich öffnende Gasaustrittsöffnungen (vorzugsweise gemäß vorstehender Beschreibung) im Bereich der Strom - Sperrkante, d.h. unmittelbar distal zur Strom - Sperrkante aber noch im Strömungsfeld des austretenden Gases angeordnet sind, welche das Abtropfen von Flüssigkeit unterstützen können.

Um ein Überströmen der Fluidstrom - Sperrkante mit in Richtung distal geöffneter Umfangsnut gut zu ermöglichen, geht die Sperrkante in Richtung proximal kontinuierlich bzw. fliesend (ohne Absatzbildung) in die Schaftaußenseite über und bildet so an dieser Axialstelle kein (nennenswertes) Strömungshindernis.

Die Erfindung wird nachstehend anhand bevorzugter Ausführungsbeispiele unter Bezugnahme auf die begleitenden Figuren näher erläutert.
- Fig. 1: zeigt die Seitenansicht einer medizinischen / chirurgischen Instrumenten-Einführhilfe gemäß einem ersten bevorzugten Ausführungsbeispiel der vorliegenden Erfindung, wobei in diesem Ausführungsbeispiel jedoch die beanspruchten Merkmale der Fluidstromsperrkante als Kreisrinne oder -nut nicht dargestellt sind, sondern weitere zusätzliche Merkmale erläutert werden,
- Fig. 2: zeigt den Längschnitt der Instrumenten - Einführhilfe längs der Schnittlinie A - A gemäß der Fig. 1,
- Fig. 3: zeigt den Querschnitt der Instrumenten - Einführhilfe längs der Schnittlinie B - B gemäß der Fig. 2,
- Fig. 4: zeigt der Längschnitt einer Instrumenten - Einführhilfe gemäß einem zweiten bevorzugten Ausführungsbeispiel der vorliegenden Erfindung, wobei an dieser Stelle bereits darauf hingewiesen wird, dass die nachfolgend beschriebenen technischen Merkmale des ersten Ausführungsbeispiels (obgleich in Fig. 3 nicht dargestellt) auch bei der Instrumenten - Einführhilfe des zweiten Ausführungsbeispiels verwirklicht sein können.

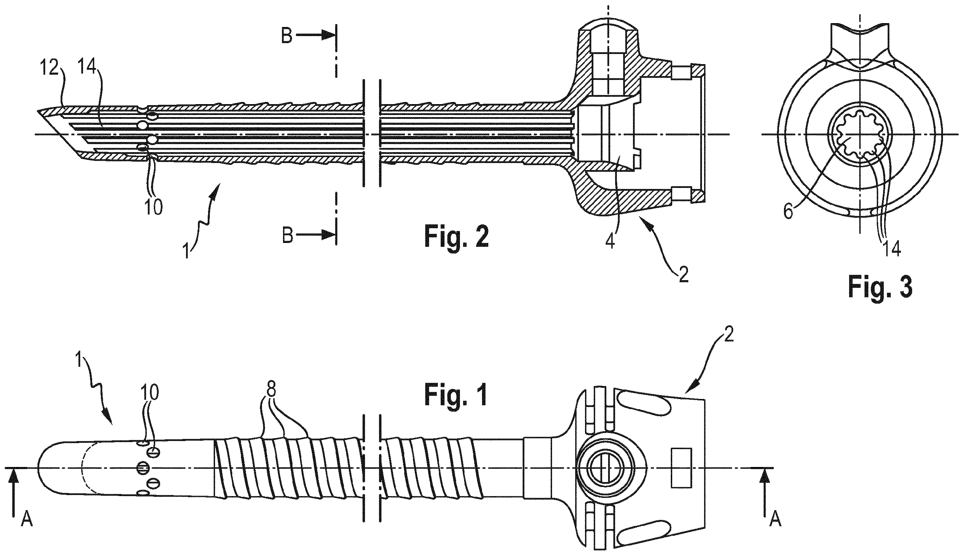

Die erfindungsgemäße Instrumenten - Einführhilfe ist vorliegend als starrer Trokar ausgebildet mit einer schaftartigen Trokarhülse 1, deren distale Stirnseite bezüglich der Trokarachse angeschrägt ist und an deren proximalem Endabschnitt ein Anschlussstutzen 2 für eine Anzahl unterschiedlicher Zusatzausstattungen wie zumindest eine Insuffliereinrichtung (nicht weiter gezeigt) angeordnet ist. Des Weiteren bildet der Anschlussstutzen 2 gemäß der Fig. 2 eine Einführöffnung 4 in axialer Verlängerung zum Schaft 1, über die chirurgische und/oder optische Einrichtungen wie ein Endoskop (nicht weiter dargestellt) in den Schaft 1 bzw. einen darin ausgebildeten Kanal 6 ein- und ausführbar sind. Der Anschlussstutzen 2 ist vorliegend auf den Schaft 1 aufgesteckt/aufgeschraubt, d.h. fest mit dem Schaft 1 verbunden. Es ist aber auch denkbar, dass Schaft 1 und Anschlussstutzen 2 stoffeinstückig ausgeformt sind. Auch ist es möglich, dass die Verbindung zwischen Schaft 1 und Anschlussstutzen 2 lösbar ist, um unterschiedliche Anschlussstutzen mit unterschiedlichen Schäften (bei gleichen Schaft - Außendurchmessern) zu kombinieren.

Gemäß dem vorliegenden Ausführungsbeispiel ist die Einführhilfe konkret ein Trokar bzw. ein Tubus (Trokarhülse). Es ist aber auch möglich, dass der gezeigte Schaft 1 aus einem biegeflexiblen Schlauchmaterial besteht, wie er im Fall einer Gastroskopie oder einer Koloskopie verwendet werden könnte. Der Schaft 1 weist gemäß der Fig. 1 außerdem außenseitig eine Anzahl von axial beabstandeten (keilförmigen) Umfangsabsätzen 8 oder eine spiralförmig sich erstreckende (keilförmige) Umfangsleiste auf, die in Richtung proximal jeweils eine Anschlagfläche bilden und in Richtung distal spitz zulaufen. Auf diese Weise kann der Tubus in die Körperhöhle eines Patienten unter Übergleiten der Umfangsabsätze 8 eingeführt werden, wobei die Umfangsabsätze aufgrund der nach proximal wirkenden Anschlagflächen ein selbständiges (unbeabsichtigtes) Herausrutschen aus der Körperhöhle blockieren bzw. behindern.

Schließlich zeigt der Schaft/Tubus 1 in dessen distalem Endabschnitt eine Anzahl von in Umfangsrichtung (gleichmäßig) beabstandeter, radial sich erstreckender Durchgangsbohrungen / -Öffnungen 10 durch die Schaftwand hindurch, welche den innerhalb des Schafts 1 ausgebildeten Einführkanal 6 (siehe Fig. 2) mit der lokalen Schaftumgebung fluidverbinden. Wie insbesondere in der Fig. 1 dargestellt ist, sind die radialen Durchgangsbohrungen 10 vorzugsweise in Axialrichtung wechselweise versetzt angeordnet und bilden so wenigstens zwei (oder mehrere) axial beabstandete Bohrungs - Umfangsreihen. Die Durchgangsbohrungen / Durchgangsöffnungen 10 sind vorliegend runde Bohrungen mit jeweils einem Durchmesser, derart, dass sich die Bohrungen 10 der beiden axial beabstandeten Reihen nicht überlappen, sodass zwischen den jeweils benachbarten Bohrungen 10 derselben Reihe wie auch zwischen zwei benachbarten Bohrungen 10 unterschiedlicher Reihen immer ein Schaftmaterialsteg vorbestimmter Stegbreite verbleibt. Dadurch bleibt die Materialschwächung des Schafts 1 in diesem Bereich gering und die Schaftstabilität weitgehend erhalten. Alternativ hierzu ist es aber auch möglich, dass sich die Durchgangsöffnungen 10 der zwei Reihen in Axialrichtung gesehen überlappen.

Wie insbesondere aus der Fig. 2 zu entnehmen ist, hat der Einführkanal 6 in einem Axialabschnitt zwischen dem distalen Schaftende und den radialen Durchgangsöffnungen 10 eine radiale Einschnürung 12 bzw. eine radial nach innen vorragende, geschlossen umlaufende Verengung, wodurch sich der Einführkanal - Durchmesser in diesem Bereich verringert, derart, dass ein in den Einführkanal 6 eingeschobenes Instrument (oder Obturator) in etwa gleitend an dieser distalen Einschnürung / Verengung 12 geführt wird. Die Einschnürung 12 erstreckt sich dabei in Axialrichtung des (Trokar-) Schafts 1 bis zu dessen distaler Stirnseite.

Schließlich weist der Schaft 1 an der Führungskanal - Innenwand zumindest eine, vorzugsweise gemäß der Fig. 3 eine Mehrzahl von in Umfangsrichtung (gleichmäßig) beabstandeter Längsnuten 14 auf, die sich vorliegend gerade in Achsrichtung erstrecken und vor Erreichen der distalen Stirnseite des Schafts 1 vorzugsweise im Bereich der Einschnürung 12 enden bzw. auslaufen. Durch das Auslaufen der Nuten 14 (etwa 2 - 3mm vor der distalen Schaftspitze) wird ein glatter Übergang erzeugt, sodass ein Instrument/Obturator klemmfrei durch den Einführkanal 6 geschoben werden kann. Die Nut/Nuten 14 sind dabei so angeordnet, dass sie die radialen Durchgangsöffnungen 10 schneiden und so eine direkte Fluidverbindung zu den Durchgangsöffnungen 10 haben. Es ist alternativ aber auch möglich, die Nuten 14 nicht gerade sondern schräg zur Schaftachse, spiralförmig oder im Zick - Zack - Kurs auszuformen. Entscheidend hierbei ist, dass die Schaftwand durch die Nut(en) 14 in diesem Bereich nicht übergebührlich geschwächt wird, um die Schaftstabilität gegenüber einem (nicht gezogenen) Standartschaft nicht nachhaltig zu verändern. Auch sollte die Nutentiefe so gewählt sein, dass der Außendurchmesser des erfindungsgemäßen Schafts in etwa dem des (nicht gezogenen) Standartschafts entspricht.

Die Funktion der erfindungsgemäßen Einführhilfe lässt sich wie Folgt zusammenfassen:
Wird die gezeigte Trokarhülse (Schaft) mit bereits eingesetztem Obturator oder chirurgischen Instrument (Optik) in eine Körperhöhle eingeführt, besteht grundsätzlich die Möglichkeit, dass Blut und/oder Gewebeflüssigkeit an der Schaftaußenseite in distale Richtung abläuft und sich an der distalen Schaftspitze / -kante tropfenförmig ansammelt. Da jedoch zur Insufflation der Körperhöhle ständig ein Gas durch einen (nicht weiter gezeigten) Ringspalt zwischen dem Instrument / Obturator und dem (Trokar-) Schaft 1 sowie hauptsächlich durch die zumindest eine Nut 14 oder die Nuten 14 in ausreichender Menge (ausreichend hohes Gasvolumen pro Zeiteinheit) bis zu den Durchgangsöffnungen 10 geleitet wird und von dort radial nach Außen ausströmt, kann (ggf. in Abhängigkeit der Ausströmgeschwindigkeit sowie des Abstands der jeweils benachbarten Auslassöffnungen 10) ein (vorzugsweise in Umfangsrichtung im wesentlichen geschlossener) eine Art Gasstrahlvorhang / -wand aufgebaut werden, der quasi als Strömungssperre/-Bremse für das Blut-/ Gewebeflüssigkeitsgemisch in Axialrichtung wirken kann (pneumatische Tropfensperre). Auf diese Weise kann bereits im Vorfeld einer Tropfenbildung aus Blut/Gewebeflüssigkeit an der distalen Schaftspitze zumindest in einem gewissen Ausmaß entgegengewirkt werden.

Wird nunmehr der Obturator oder das chirurgische Instrument (Optik, Endoskop) aus der Einführhilfe, d.h. dem (Trokar-) Schaft 1 zurückgezogen, kann der Zustand eintreten, dass ein wahrscheinlich noch vorhandener Rest - Fluidtropfen in den Ringspalt zwischen Obturator/Instrument/Optik und Torkarhülse eingesogen wird. Jedoch wandert in diesem Fall die Tropfenflüssigkeit nicht über die gesamte Kanalinnenwand sondern lagert sich vorzugsweise im Wesentlichen in den Längsnuten 14 an. Wird folglich das Instrument/Optik erneut in Richtung distal verschoben, kann es möglich sein, die Flüssigkeit weitgehend in der/den Nuten 14 zu halten, sodass die Optik nicht bzw. nicht spürbar verschmutzt wird. Dies bedeutet aber auch, dass die vorstehend erwähnte optionale Funktion der Radial-Gasauslassöffnungen als (pneumatische) Topfensperre/-bremse prinzipiell nicht notwendig ist, da distal sich ansammelnde Fluidtropfen ggf. nicht/nur geringfügig in den Ringspalt gelangen.

Grundsätzlich ist jedoch die Ausbildung der zumindest einen Längsnut 14 an der Innenseite des Einführkanals 6 vorzugsweise in Verbindung mit den radialen Gasauslassöffnungen 14 einfach in der Herstellung und daher vorteilhaft speziell für Einweg - Trokare beispielsweise aus Kunststoff geeignet. Durch sie wird eine wirksame Verbesserung des Gasdurchflusses bei im Wesentlichen unverändertem Außendurchmesser erreicht.

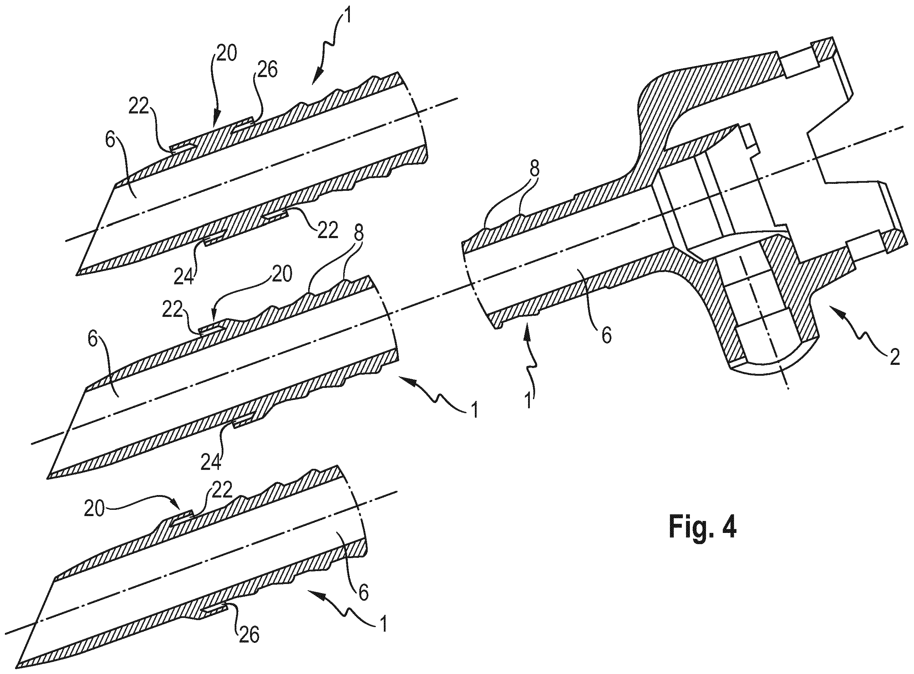

Die Fig. 4 zeigt ein zweites Ausführungsbeispiel einer erfindungsgemäßen Einführhilfe dieser Gattung, wobei nur jene technischen Merkmale dargestellt sind, durch welche sich das zweite Ausführungsbeispiel vom zuvor beschrieben ersten Ausführungsbeispiel der Erfindung unterscheidet. Es besteht also prinzipiell die Möglichkeit, alle oder ausgewählte Merkmale des ersten Ausführungsbeispiels, wie die inneren Längsnut(en) und/oder die Radial-Gasauslassöffnungen auch bei der Einführhilfe gemäß der Fig. 4 vorzusehen.

Die medizinische Instrumenten - Einführhilfe gemäß der Fig. 4 besteht ebenfalls aus dem Schaft 1 sowie dem distal daran angeschlossenen/ausgebildeten Anschlussstutzen 2. Im Schaft 1 ist der Einführ- oder Arbeitskanal 6 für medizinische Instrumente (nicht gezeigt) angedeutet (ggf. mit Längsnut(en) ausgestattet), der sich am distalen Ende des Schafts 1 zur Atmosphäre hin öffnet. Obgleich nicht weiter gezeigt, kann der Schaft 1 mit radialen Auslassöffnungen/-bohrungen und/oder inneren Kanallängsnut(en) gemäß dem ersten bevorzugten Ausführungsbeispiel ausgebildet sein.

Am Außenumfang sowie in einem axialen Mittenabschnitt des Schafts 1, vorzugsweise im Bereich seines distalen Endes ist eine vorliegend geschlossen umlaufende, radial vorragende Fluidstrom-Sperrkante 20 angeordnet/ausgeformt, die sich (wie in der Fig. 4 gezeigt wird) in ihrer Form und/oder ihrer radialen Erstreckung von jenen (schuppenförmigen) Vorsprungskanten 8 unterscheidet, die im Axialabstand zueinander ebenfalls am Schaftaußenumfang vorzugsweise ausgebildet sind, die jedoch nur die Aufgabe eines Haltens des Schafts 1 im Patientenhohlraum haben, herab laufendes Fluid jedoch nicht (nachhaltig) aufhalten können.

Die Fluidstrom-Sperrkante 20 hat vorliegend im Querschnitt eine L-, U-, V-, X- oder H-Form, wodurch die Sperrkante 20 zwischen sich und der Schaftaußenseite zumindest eine umlaufende Nut oder Rinne 22 bildet, die axial in Richtung distal und/oder proximal offen ist. Aus der ausgewählten Öffnungsrichtung der Umfangs- Nut/Rinne ergeben sich unterschiedliche Sperrwirkungen/Effekte, wie sie nachfolgend beschrieben werden:

### 1. Umlaufende Rinne mit Öffnung in Richtung distal

In diesem Fall bildet die Fluidstrom-Sperrkante 20 eine freie, umlaufende (Abriss-) Kante 24 unter Ausbildung einer axialen Umlaufnut 22 mit Nutenöffnung in Richtung distalem Schaftende. Diese freie (Strömungsabriss-) Kante 24 dient als Tropfen-Ansammelkante, an der sich herab laufende Flüssigkeit vorzugsweise tropfenförmig ansammelt und bei Erreichen einer bestimmten Tropfengröße (Tropfengewicht) eigenständig abtropft. Dieser Abtropfvorgang kann ggf. unterstützt werden durch die radialen Gasauslassöffnungen 10, die bevorzugt im unmittelbaren axialen Anschluss in Richtung distal zur Fluidstrom - Sperrkante 20 platziert sein können, (derart, dass sich die Sperrkante 20 und insbesondere die Abrisskante 24 noch im Ausströmfeld der Auslassöffnungen 10 befindet).

Darüber hinaus ist es optional möglich, die Spaltbreite der Nut 22 so zu wählen, dass hierdurch eine Art Kapillarwirkung auf das Fluid erzeugbar ist, um das Fluid entgegen seiner Stromrichtung in die Nut quasi einzusaugen und so an einer Weiterströmung zu hindern.

### 2. Umlaufende Rinne mit Öffnung in Richtung proximal

In diesem Fall bildet die Fluidstrom-Sperrkante 20 eine freie, umlaufende (Einleit-) Kante 26 unter Ausbildung einer axialen Umlaufnut 22 mit Nutenöffnung in Richtung proximalem Schaftende. Diese Nut/Rinne 22 ist demnach in Richtung entgegen der Fluidstromrichtung (umlaufend) geöffnet und dient somit als eine Art Auffang-/Sammelrinne für das herab strömende Fluid. Die Nutentiefe dieser nach proximal geöffneten Nut 22 ist daher so gewählt, dass genug Flüssigkeit darin aufnehmbar ist, die im Rahmen einer Patientenbehandlung erfahrungsgemäß entsteht.

Auch hier ist es optional möglich, die Spaltbreite der Nut 22 so zu wählen, dass eine Art Kapillarwirkung auf das Fluid erzeugbar ist, um das Fluid in die Nut quasi einzusaugen und so ein unbeabsichtigtes Austreten insbesondere bei Erreichen des maximalen Nuten - Füllstands zu verhindern.

Abschließend sei erwähnt, dass nur eine Rinne (in diesem Fall in L-, U-oder V-Form) oder zwei Rinnen mit Rinnenöffnungen in unterschiedliche Axialrichtungen (in diesem Fall in X- oder H-Form) vorgesehen sein können. Auch besteht durchaus die Möglichkeit, zwei (oder mehrere) Rinnen mit gleicher Öffnungsausrichtung im Axialabstand zueinander anzuordnen.

Zusammenfassend wird eine medizinische Einführhilfe für chirurgische Instrumente und/oder Optiken offenbart mit einem einen Einführkanal 6 ausbildenden Schaft 1, an dessen Innenwand zumindest eine in Kanallängsrichtung verlaufende oder längsorientierte Nut 14 ausgeformt ist. Zusätzlich oder alternativ wird eine medizinische Einführhilfe offenbart, die in ihrem distalen Schaft - Endabschnitt (axialer Mittenabschnitt vorzugsweise im distalen Endbereich) eine radial auswärts sich erstreckende, vorzugsweise geschlossen umlaufende Fluidstrom - Sperrkante hat. Diese bildet zumindest eine axial sich öffnende Kreisrinne oder -nut mit Öffnungsrichtung nach distal und/oder proximal.

## Patentansprüche

1. Medizinische Einführhilfe für chirurgische Instrumente und/oder Optiken, mit einem einen Einführkanal (6) ausbildenden Schaft (1), wobei an der Innenwand des Einführkanals (6) zumindest eine in Kanallängsrichtung verlaufende oder längsorientierte Nut (14) ausgeformt ist, **dadurch gekennzeichnet, dass** der Schaft (1) in seinem axialen Mitten- und/oder distalen Endabschnitt zumindest eine radial auswärts sich erstreckende, vorzugsweise geschlossen umlaufende Fluidstrom-Sperrkante (20) hat, wobei die zumindest eine Fluidstrom-Sperrkante (20) zumindest eine axial sich öffnende Kreisrinne oder -nut (22) bildet mit Öffnungsrichtung nach distal und/oder proximal.

2. Medizinische Einführhilfe nach Anspruch 1, **dadurch gekennzeichnet, dass** eine Mehrzahl von Nuten (14) vorzugsweise in gleichem Umfangsabstand zueinander an der Innenwand der Einführkanals (6) ausgeformt ist.

3. Medizinische Einführhilfe nach Anspruch 1 oder 2, **dadurch gekennzeichnet, dass** die zumindest eine Nut (14) oder die Mehrzahl von Nuten (14) vor Erreichen des distalen Schaftendes auslaufen.

4. Medizinische Einführhilfe nach Anspruch 3, **dadurch gekennzeichnet, dass** im distalen Endabschnitt des Schafts (1) eine radiale Einschnürung (12) oder Einziehung den Einführkanal (6) über dessen gesamten Umfang verengt, wobei die zumindest eine Nut oder die Mehrzahl von Nuten (14) vorzugsweise proximal zur Einschnürung oder Einziehung (12) oder in deren axialen Mittenabschnitt auslaufen.

5. Medizinische Einführhilfe nach einem der vorstehenden Ansprüche, **gekennzeichnet durch** eine Anzahl von radialen Durchgangsbohrungen (10) im distalen Endabschnitt des Schafts (1) sowie vorzugsweise in einem proximalen Abstand zur radialen Einschnürung (12) nach Anspruch 4, welche den Einführkanal (6) mit der lokalen Schaftumgebung radial verbinden.

6. Medizinische Einführhilfe nach Anspruch 5, **dadurch gekennzeichnet, dass** die Anzahl von radialen Durchgangsbohrungen (10) in Umfangsrichtung voneinander vorzugsweise gleichmäßig beabstandet und weiter vorzugsweise so ausgerichtet und/oder platziert sind, dass sie in die Nuten (14) an der Innenwand des Einführkanals (6) münden oder sich mit diesen schneiden.

7. Medizinische Einführhilfe nach Anspruch 5 oder 6, **dadurch gekennzeichnet, dass** die Durchgangsbohrungen (14) in Axialrichtung vorzugsweise wechselweise voneinander versetzt sind.

8. Medizinische Einführhilfe nach Anspruch 7, **dadurch gekennzeichnet, dass** sich die axial versetzt zueinander angeordneten Durchgangsbohrungen (10) in Umfangsrichtung jeweils überlappen.

9. Medizinische Einführhilfe nach Anspruch 1, **dadurch gekennzeichnet, dass** die zumindest eine Fluidstrom-Sperrkante (20) eine umlaufende freie Stromabtropfkante (24) im Fall einer nach distal geöffneten Umfangsnut (22) und/oder eine umlaufende freie Stromeinleitkante (26) im Fall einer nach proximal geöffneten Umfangsnut (22) bildet.

## Claims

1. A medical insertion aid for surgical instruments and/or optical systems comprising a shank (1) forming an insertion duct (6), wherein at least one groove (14) extending in the longitudinal duct direction or being longitudinally orientated is formed at the inner wall of the insertion duct (6), **characterized in that** the shank (1) in its axial central and/or distal end portion has at least one radially outwardly extending, preferably closed peripheral fluid flow inhibiting edge (20), wherein the at least one fluid flow inhibiting edge (20) forms at least one axially opening circular channel or groove (22) with the opening being directed distally and/or proximally.

2. The medical insertion aid according to claim 1, **characterized in that** a plurality of grooves (14) is formed preferably at equal circumferential distance from each other at the inner wall of the insertion duct (6).

3. The medical insertion aid according to claim 1 or 2, **characterized in that** the at least one groove (14) or the plurality of grooves (14) run out before they reach the distal shank end.

4. The medical insertion aid according to claim 3, **characterized in that** in the distal end portion of the shank (1) a radial constriction (12) or contraction narrows the insertion passage (6) over the entire periphery thereof, the at least one groove or the plurality of grooves (14) running out preferably proximally relative to the constriction or contraction (12) or in the axial central portion thereof.

5. The medical insertion aid according to any one of the preceding claims, **characterized by** a number of radial through bores (10) in the distal end portion of the shank (1) as well as preferably at a proximal distance from the radial constriction (12) according to claim 4 which radially connect the insertion duct (6) to the local shank environment.

6. The medical insertion aid according to claim 5, **characterized in that** the number of radial through bores (10) are preferably evenly spaced from each other in the peripheral direction and further preferably are orientated and/or placed so that they open into the grooves (14) at the inner wall of the insertion duct (6) or intersect the same.

7. The medical insertion aid according to claim 5 or 6, **characterized in that** the through bores (14) are preferably alternately offset from each other in the axial direction.

8. The medical insertion aid according to claim 7, **characterized in that** the respective axially offset through bores (10) are overlapping in the peripheral direction.

9. The medical insertion aid according to claim 1, **characterized in that** the at least one fluid flow inhibiting edge (20) forms a peripheral free flow drain edge (24) in the case of a distally opened peripheral groove (22) and/or a peripheral free flow introducing edge (26) in the case of a proximally opened peripheral groove (22).

## Revendications

1. Auxiliaire d'insertion médical pour des instruments chirurgicaux et/ou des optiques, avec une tige (1) réalisant un canal d'insertion (6), dans lequel, au niveau de la paroi interne du canal d'insertion (6), est formée au moins une rainure (14) s'étendant dans la direction longitudinale du canal ou orientée longitudinalement, **caractérisé en ce que** la tige (1) a, dans sa section d'extrémité axiale médiane et/ou distale, au moins un bord de blocage de flux de fluide (20) s'étendant radialement vers l'extérieur, de préférence périphérique fermé, dans lequel l'au moins un bord de blocage de flux de fluide (20) forme au moins un sillon ou une rainure (22) circulaire s'ouvrant axialement avec une direction d'ouverture distale et/ou proximale.

2. Auxiliaire d'insertion médical selon la revendication 1, **caractérisé en ce qu'**une pluralité de rainures (14) sont formées de préférence à une même distance périphérique les unes des autres au niveau de la paroi intérieure du canal d'insertion (6).

3. Auxiliaire d'insertion médical selon la revendication 1 ou 2, **caractérisé en ce que** l'au moins une rainure (14) ou la pluralité de rainures (14) se terminent avant d'atteindre l'extrémité de tige distale.

4. Auxiliaire d'insertion médical selon la revendication 3, **caractérisé en ce que,** dans la section d'extrémité distale de la tige (1), un étranglement radial (12) ou un retrait du canal d'insertion (6) se resserre sur toute sa périphérie, dans lequel l'au moins une rainure ou la pluralité de rainures (14) se terminent de préférence de manière proximale à l'étranglement ou au retrait (12) ou dans leur section médiane axiale.

5. Auxiliaire d'insertion médical selon l'une des revendications précédentes, **caractérisé par** un nombre d'alésages traversants (10) radiaux dans la section d'extrémité distale de la tige (1) et de préférence à une distance proximale par rapport à l'étranglement radial (12) selon la revendication 4, qui relie radialement le canal d'insertion (6) à l'environnement de tige local.

6. Auxiliaire d'insertion médical selon la revendication 5, **caractérisé en ce que** nombre d'alésages traversants (10) radiaux dans la direction périphérique sont distants de préférence uniformément les uns des autres et de manière davantage préférée, sont orientés et/ou placés de sorte qu'ils débouchent dans les rainures (14) au niveau de la paroi intérieure du canal d'insertion (6) ou coupent celui-ci.

7. Auxiliaire d'insertion médical selon la revendication 5 ou 6, **caractérisé en ce que** les alésages traversants (14) sont décalés les uns des autres en direction axiale de préférence alternativement.

8. Auxiliaire d'insertion médical selon la revendication 7, **caractérisé en ce que** les alésages traversants (10) disposés axialement de façon décalée les uns par rapport aux autres se chevauchent respectivement dans la direction périphérique.

9. Auxiliaire d'insertion médical selon la revendication 1, **caractérisé en ce que** l'au moins un bord de blocage de flux de fluide (20) forme un bord d'égouttage de flux (24) périphérique libre dans le cas d'une rainure périphérique (22) ouverte dans une direction distale et/ou un bord d'amorçage de flux (26) périphérique libre dans le cas d'une rainure périphérique (22) ouverte dans une direction proximale.
